## Europäisches Patentamt

## European Patent Office

## Office européen des brevets

(19)

(11) Numéro de publication : **0 187 283 B1**

(12) **FASCICULE DE BREVET EUROPÉEN**

(45) Date de publication du fascicule du brevet :
26.04.89

(51) Int. Cl.⁴ : **A 61 B 17/58**

(21) Numéro de dépôt : 85115629.9

(22) Date de dépôt : 09.12.85

(54) Appareil pour repérer in situ les trous transversaux d'une broche creuse implantée dans le canal médullaire, pour la contention des fragments d'un os fracturé.

(30) Priorité : 26.12.84 FR 8419956
16.01.85 FR 8500881

(43) Date de publication de la demande :
16.07.86 Bulletin 86/29

(45) Mention de la délivrance du brevet :
26.04.89 Bulletin 89/17

(84) Etats contractants désignés :
CH DE FR GB IT LI

(56) Documents cités :
EP-A- 0 086 883
FR-A- 2 425 840
FR-A- 2 524 296
GB-A- 4 033 043
US-A- 2 666 430
US-A- 2 697 433

(73) Titulaire : Nivarox-FAR S.A.
Avenue du Collège 10
CH-2400 Le Locle (CH)

(72) Inventeur : Buzzi, Carlo
Stauffacherstrasse 98
CH-8004 Zürich (CH)
Inventeur : de Couet, Alexandre
Weinbergstrasse 115
CH-8006 Zürich (CH)
Inventeur : Hamid, Mohamed
18, rue du Canal de la Marne
F-67300 Schiltigheim (FR)

(74) Mandataire : Caron, Gérard et al
ICB Ingénieurs Conseils en Brevets SA Passage
Max. Meuron 6
CH-2001 Neuchâtel (CH)

## Description

La présente invention est relative à un appareil destiné à être utilisé au cours de la fixation d'une broche dans un os fracturé.

Les interventions d'osthéosyntèse sont réalisées, à l'heure actuelle, à l'aide d'éléments rigides de renforcement qui sont associés à l'os fracturé afin de le renforcer et de rendre au patient, le plus rapidement possible, ses facultés de mouvement. Ces éléments de renforcement sont constitués par exemple par des plaques, fixées sur la face extérieure de l'os de part et d'autre de la fracture, ou par des broches creuses qui sont destinées à être insérées dans le canal médullaire de l'os. Ces derniers éléments sont utilisés de plus en plus souvent, car ils sont le moins traumatisants pour le patient et permettent une rigidification de l'os fracturé, la broche résistant non seulement aux efforts de compression ou de traction, mais également à la torsion. C'est cette dernière propriété des broches qui ne peut être obtenue que si elle est bloquée non seulement longitudinalement, mais également en rotation autour de son axe notamment dans certains types de fractures dites « basses » ou « hautes » (fractures diaphyso-épiphysères). Par conséquent, si la broche doit résister à la torsion, il est nécessaire de poser des vis transversales qui traversent diamétralement l'os ainsi qu'une ou plusieurs paires de trous alignés prévus notamment près de l'extrémité distale de la broche. Cette pratique est surtout appliquée dans le cas où l'os fracturé est le fémur ou le tibia.

Les broches sont connues en soi. Elle présentent généralement une section de forme approximative en trèfle, l'une des extrémités étant légèrement évasée et munie d'un filetage intérieur, l'autre extrémité étant légèrement effilée pour faciliter l'insertion dans le canal médullaire. En outre, suivant le cas d'application, broche peut être légèrement courbée le long de son axe longitudinal et elle présente longitudinalement une fente sur toute sa longueur qui lui confère une certaine souplesse en flexion, ce qui permet, lors de l'insertion, une adaptation parfaite à la forme du canal médullaire. Bien entendu, le chirurgien dispose d'un choix parmi plusieurs longueurs et plusieurs diamètres qui sont fonction de l'os traité et de la taille du membre du patient.

Selon une technique qui est actuellement employée, décrite notamment dans le document US-A-4 418 422, le chirurgien procède d'abord à la pose de la broche selon une procédure connue puis s'emploie à localiser les trous transversaux se trouvant à l'extrémité distale de la broche. A cet effet, il a à sa disposition un appareil de localisation à rayons X au moyen duquel il peut pointer sur la peau du membre l'endroit où l'axe des trous traverse cette peau puis après avoir pratiqué une incision, il perce un trou à l'aide d'une perceuse en passant par les trous de la broche. Enfin, la vis peut être posée.

Ce processus nécessite donc un travail aux rayons X auquel sont exposés aussi bien le patient que le personnel traitant. L'opération de localisation prenant à peu près deux heures, il faut compter sur une exposition d'à peu près 400 à 1'000 REM par intervention. Cette dose importante, à laquelle est exposé le personnel traitant chaque fois qu'une opération est effectuée, fait hésiter jusqu'ici les chirurgiens à employer cette méthode, ceux-ci préférant soit utiliser des plaques soit des broches dépourvues de vis transversales de renforcement.

La durée prolongée de l'emploi de l'appareil au cours d'une intervention le bloque naturellement pour les interventions qui sont réalisées en même temps dans un bloc opératoire d'hôpital autre que celui dans lequel a lieu l'intervention considérée. Ceci nécessite une programmation très serrée des interventions ce qui est peu souhaitable notamment s'il y a des urgences.

Selon une autre technique, décrite notamment dans le document FR-A-2 524 296, on utilise un dispositif de visée comportant une tête de visée qui présente deux perçages. Avant d'implanter la broche, on monte le dispositif de visée sur la broche et on aligne les perçages sur les trous de la broche. On pose ensuite la broche. Celle-ci se déformant lors de la pose, on corrige la position de la tête de visée à l'aide d'un appareil de localisation à rayons X.

L'invention a donc pour but de fournir un appareil de repérage in situ des trous transversaux d'une broche creuse implantée dans le canal médullaire pour la contention des fragments d'un os fracturé qui soit conçu de manière à éviter tout emploi de rayons X au cours du repérage.

Selon l'invention, l'appareil pour repérer in situ les trous transversaux d'une broche creuse implantée dans le canal médullaire d'un os pour la contention de fragments de celui-ci après une fracture, ladite broche étant pourvue à son extrémité proximale de moyens de fixation pour ledit appareil, tandis que le ou les trous à repérer sont prévus au moins à son extrémité distale, cet appareil comprenant :

— un support destiné à être rapporté sur ladite broche à l'aide desdits moyens de fixation ;

— un guide de perçage définissant un axe devant être mis en coïncidence avec l'axe du trou à repérer pour permettre le perçage transversal de l'os exactement en alignement avec ce trou ;

— une monture solidaire dudit support et portant ledit guide de perçage, ladite monture comprenant au moins une première articulation permettant une rotation dudit guide de perçage autour de ladite broche dans un plan contenant l'axe du trou et une deuxième articulation permettant une translation de l'axe du guide de perçage parallèle à lui-même ;

ledit appareil étant caractérisé en ce qu'il comprend en outre :

— des premiers moyens palpeurs destinés à être insérés dans ladite broche en s'orientant en fonction de la direction de l'axe du trou, et des premiers moyens indicateurs associés auxdits premiers moyens palpeurs et disposés à l'extérieur de ladite broche pour indiquer l'angle α formé par l'axe dudit trou et l'axe dudit guide de perçage, lorsque lesdits premiers moyens palpeurs sont insérés dans ladite broche,

— des seconds moyens palpeurs destinés à émettre un rayonnement électromagnétique à partir dudit trou vers le guide de perçage et des seconds moyens indicateurs associés audit guide de perçage pour indiquer la distance entre l'axe du trou et l'axe du guide de perçage,

— des moyens de réglage pour amener l'axe dudit guide de perçage en coïncidence avec l'axe du trou en fonction des informations angulaire et de distance fournies successivement par les premiers et seconds moyens palpeurs.

Grâce à ces caractéristiques, le repérage peut être réalisé en quelques minutes seulement sans emploi de rayons X ce qui constitue un gain de temps appréciable notamment chez les polytraumatisés. En effet, les moyens de mesure peuvent être mécaniques et/ou du type à rayonnement électromagnétique dont la longueur d'onde est située dans une plage qui rend ce rayonnement non traumatisant tant pour le personnel de service que pour le patient.

L'invention sera mieux comprise à la lecture de la description qui va suivre de plusieurs modes de réalisation de l'invention, description qui est faite en référence aux dessins annexés sur lesquels :

— les figures 1 et 1A montrent une représentation schématique de la position de l'appareil suivant l'invention au cours d'une intervention de contention d'une fracture du fémur ;

— les figures 2 et 2A montrent des vues analogues à celles des figure 1 et 1A, montrant le cas de la contention d'une fracture du tibia ;

— la figure 3 représente un diagramme permettant l'analyse du principe qui est à la base de la présente invention ;

— la figure 4 est une représentation schématique en perspective, à plus grande échelle par rapport aux figures 1 et 2, d'un premier mode de réalisation de l'invention ;

— la figure 5 est une vue partielle en perspective éclatée de la partie proximale de l'appareil suivant l'invention ;

— les figures 5A et 5B montrent par des vues en coupe un détail de l'appareil représenté aux figures 4 et 5 ;

— les figures 6 et 7 sont de vues en perspective montrant, pour le cas de la contention d'une fracture du tibia, l'implantation de l'appareil suivant l'invention, les deux vues étant prises respectivement selon des angles différents ;

— la figure 8 est une vue en perspective schématique des premiers moyens palpeurs utilisés dans l'appareil suivant l'invention ;

— la figure 9 montre les seconds moyens palpeurs de cet appareil ;

— la figure 10 est une représentation en perspective d'une variante de l'appareil ;

— les figures 11 et 12 représentent une autre forme de réalisation de l'invention, respectivement avant et après le réglage ;

— les figures 13A à 13D montrent quatre variantes des premiers moyens palpeurs, respectivement en a et b ;

— les figures 14A à 14D montrent quatre variantes des seconds moyens palpeurs ;

— les figures 15A à 15C concernent d'autres variantes de ces seconds moyens palpeurs.

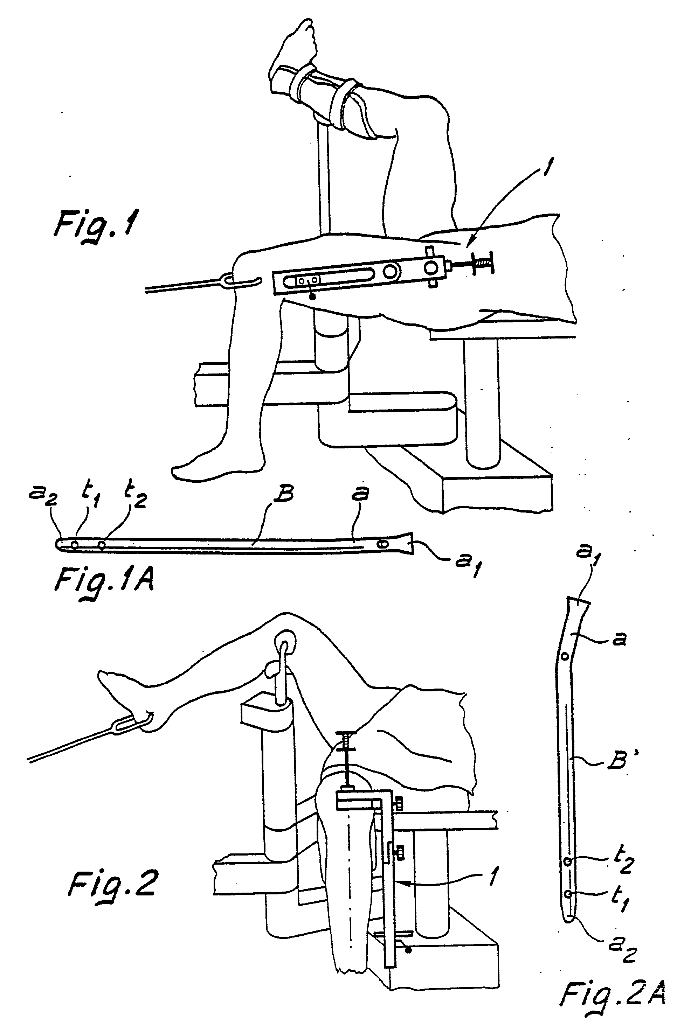

Les figures 1 et 1A représentent le cas d'une intervention d'ostéosynthèse sur le fémur, la figure 1 montrant la position de l'appareil suivant l'invention, désignée globalement par la référence 1, lors du repérage d'un trou transversal pratiqué dans une broche que l'on suppose déjà être mise en place dans le canal médullaire du fémur.

La figure 1A montre un exemple d'une broche, par ailleurs connue, pouvant être utilisée pour une intervention d'ostéosynthèse à laquelle s'applique l'appareil suivant l'invention. On voit cette broche B est formée par une tige qui est réalisée en un métal inerte aux humeurs et tissus humains. Cette tige est creuse est présente une section à peu près en forme de trèfle lui conférant une certaine souplesse de manière que la tige puisse s'adapter parfaitement à la forme du canal médullaire lorsqu'elle est mise en place dans l'os. On notera donc dès à présent que non seulement la tige n'a pas une forme rectiligne, mais que de plus au cours de sa mise en place dans l'os elle admettra une certaine déformation qui n'est pas connue à priori.

La broche B présente une extrémité proximale a pourvue d'une embouchure a1 qui est filetée intérieurement. Lors de son insertion, cette extrémité proximale reçoit tout d'abord une enclume qui est vissée dans le trou fileté et sur laquelle le praticien peut frapper pour faciliter l'introduction.

A son extrémité opposée a2, la broche B comporte deux trous transversaux t1 et t2 qui sont destinés à recevoir des vis de blocage (non représentées sur les figures) qui sont en général livrées avec la broche B.

Bien entendu les broches sont fournies en différentes dimensions de longueur et de diamètre comme cela est bien connu des spécialistes.

Les figures 2 et 2A représentent le cas d'une intervention d'ostéosynthèse sur le tibia, la figure 2A montrant la forme d'une broche B' utilisée dans ce cas. On voit que cette broche présente une extrémité proximale recourbée avec une embouchure comme dans le cas de la broche destinée au fémur. En outre, à l'extérieur du coude, cette broche B' comporte un trou de passage qui n'est pas visible sur le dessin. Pour le reste, les broches B et B' sont de forme identique.

Il est également à noter que lors de l'insertion des broches dans les os à contenir, elles subissent une déformation en torsion, la broche « se vissant » en quelque sorte dans le canal médullaire de par sa forme extérieure, ce qui augmente notablement la rigidité de l'ensemble constitué par la broche et l'os.

En d'autres termes, les trous t1 et t2 qu'il s'agit de repérer ne conservent, par rapport à l'embouchure, ni la position axiale, ni la position dans le plan radial par rapport à l'embouchure, ce qui complique évidemment l'opération de repérage, étant entendu qu'après l'insertion les trous ne sont plus visibles et que l'on souhaite éviter tout emploi de rayons X pour les localiser.

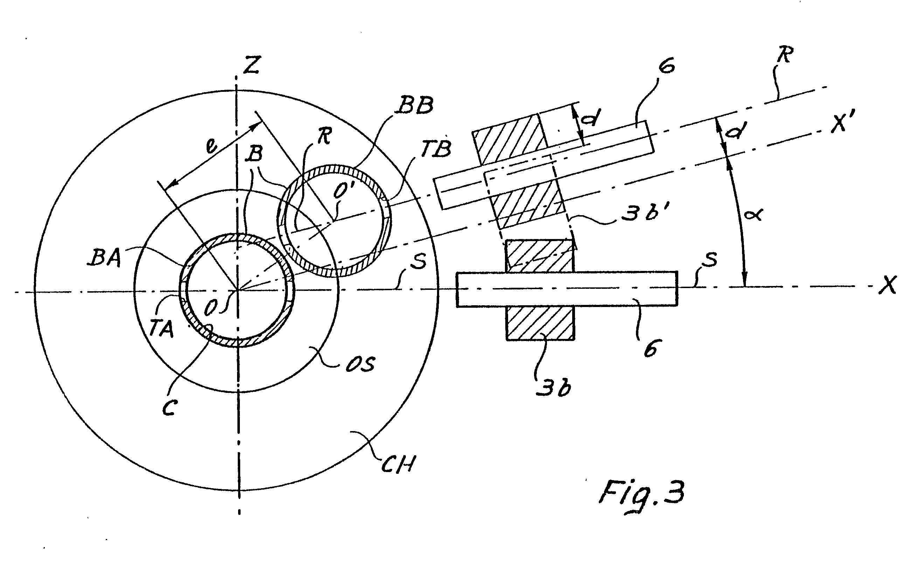

On va maintenant se référer à la figure 3 qui représente le principe de fonctionnement de l'appareil suivant l'invention, la représentation s'appliquant indifféremment aux interventions des figures 1 et 2 et bien entendu aux autres interventions pouvant être effectuées par ostéosynthèse.

Cette figure représente schématiquement l'os OS entouré de la chair CH et pourvu d'un canal médullaire C.

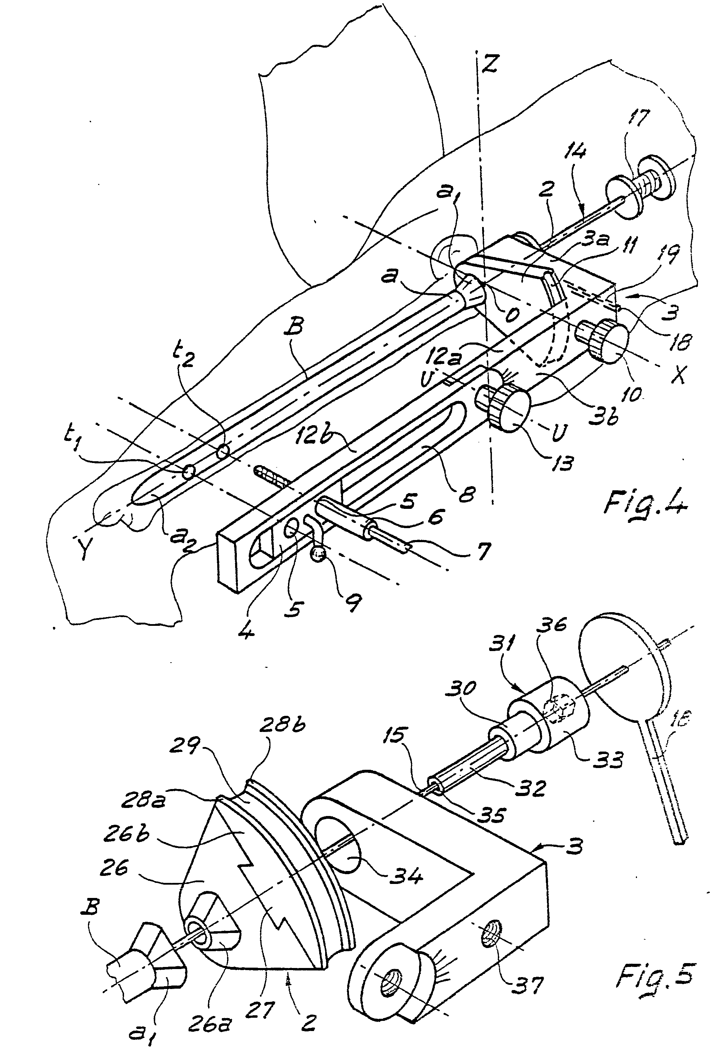

Cependant, avant de décrire en détail la figure 3, on se reportera déjà à la figure 4 pour connaître les différents organes principaux de l'appareil suivant l'invention qui, comme on peut le constater, est utilisé ici pour la contention de la fracture d'un os de fémur. On reconnait une broche B avec son extrémité proximale a et son extrémité distale a2, les deux trous transversaux t1 et t2 ainsi que l'embouchure a 1 pourvue de son filetage intérieur. C'est sur cette embouchure qu'est vissé un support 2 qui constitue l'organe de l'appareil restant fixe pendant le repérage. Ce support qui, dans l'exemple de réalisation représenté, a la forme d'un secteur de cercle, définit un plan XOZ (ici vertical) dans lequel est situé un point O considéré comme l'origine du système de coordonnées XYZ et qui est un système de référence dans la description qui va suivre. L'origine O de ce système de coordonnées est situé sur l'axe OY constituant en ce point la tangente à l'axe de la broche B qui, comme indiqué déjà ci-dessus, n'est pas une droite, mais une ligne plus ou moins courbe, suivant la forme de la broche à l'origine et également en fonction des déformations que cette broche subit lors de son insertion dans l'os.

L'appareil comporte également une équerre de réglage 3 qui est articulée sur le support 2 autour de l'axe OY.

L'équerre 3 comporte une première branche 3a s'étendant perpendiculairement à l'axe OY ainsi qu'une seconde branche 3b qui s'étend parallèlement à cet axe le long de la broche B lorsque l'appareil est en place pour la mesure.

Sur la branche 3b est monté mobile le long de son axe longitudinal un coulisseau 4 muni de deux passages transversaux 5 destinés à recevoir un guide de perçage 6. Ce dernier est dimensionné pour pouvoir recevoir un foret 7 de perçage adaptable sur une perceuse chirurgicale (non représentée). Le coulisseau 4 peut se déplacer dans une glissière longitudinale 8 de la branche 3b tout en pouvant être bloquée dans cette glissière par un mécanisme de serrage (non représenté) que l'on peut actionner à l'aide d'une manette 9.

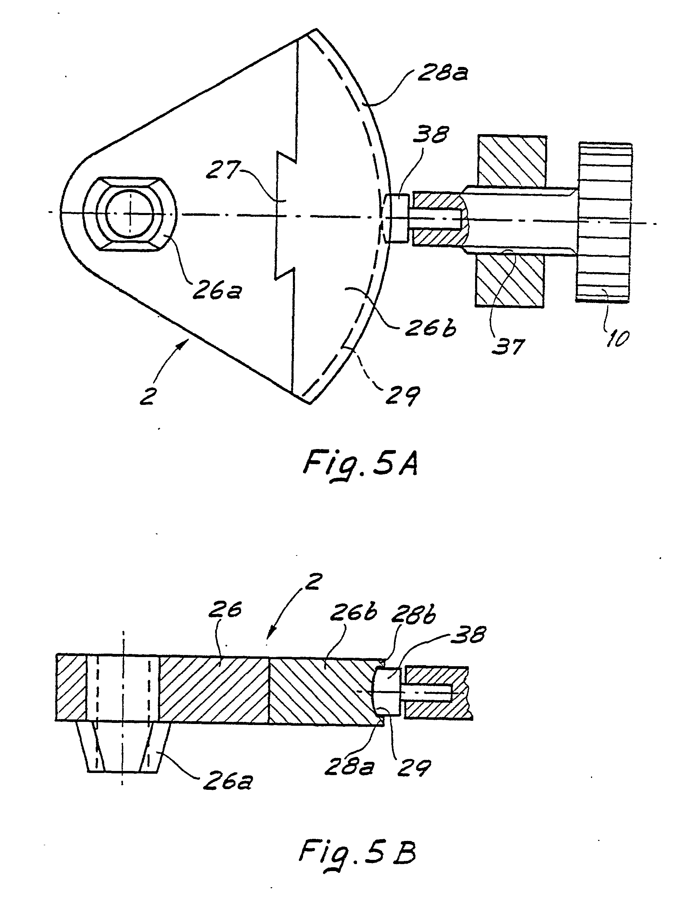

L'équerre 3 qui est donc articulée autour de l'axe OY sur le support 2 peut être bloqué par rapport à celui-ci grâce à une vis 10 qui agit sur le bord circulaire 11 du support 2 (voir également les figures 5A et 5B sur lesquelles on reviendra par la suite). En outre, la branche 3b est réalisée sous la forme d'une genouillère et est donc divisée elle-même en une première partie 12a proche du bras 3a ainsi qu'une partie 12b dans laquelle est prévue la glissière 8, l'articulation de la genouillère (axe U-U) pouvant être bloquée par une vis 13.

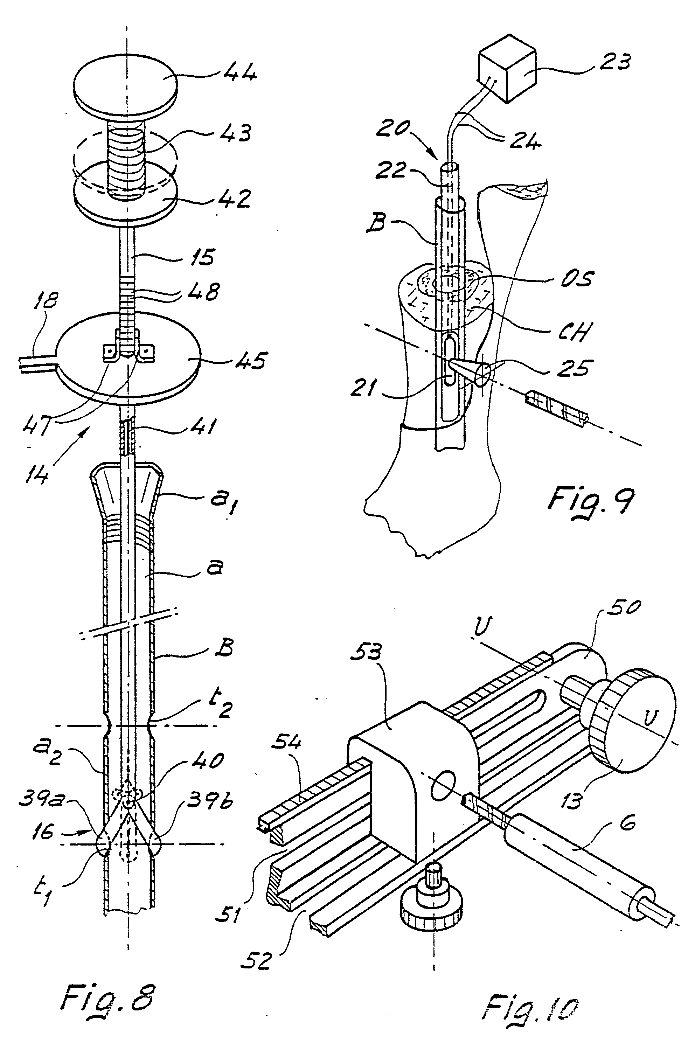

L'appareil comporte également des premiers moyens palpeurs 14 formés essentiellement d'une tige 15 qui peut être insérée dans l'alésage de la broche B et qui porte à son extrémité avant un palpeur 16 (figure 8) et à son extrémité opposée un actionneur 17, ces organes étant décrits plus loin à propos de la figure 8. Il suffit pour l'instant de noter que ces premiers moyens palpeurs 14 sont pourvus d'un index 18 qui est destiné à coopérer avec un repère 19 prévu sur l'équerre 3 et plus précisément sur la branche 3a de celui-ci à la face qui n'est pas visible sur la figure 4. Toutefois, on peut apercevoir ce repère 19 par exemple sur les figures 5, 7 et 11, l'index 18 étant à son tour le mieux visible sur la figure 5.

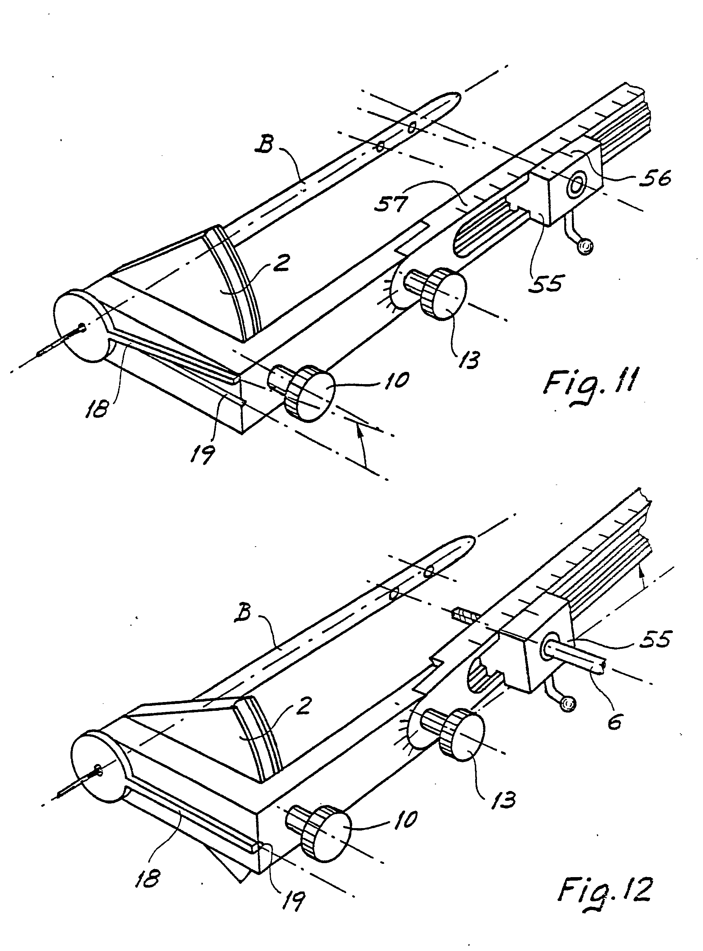

Enfin, il y a lieu à ce stade de la description de se reporter à la figure 9 qui représente un exemple de seconds moyens palpeurs 20 destinées également à être insérés dans la broche B et agissant en coopération avec le guide de perçage 6.

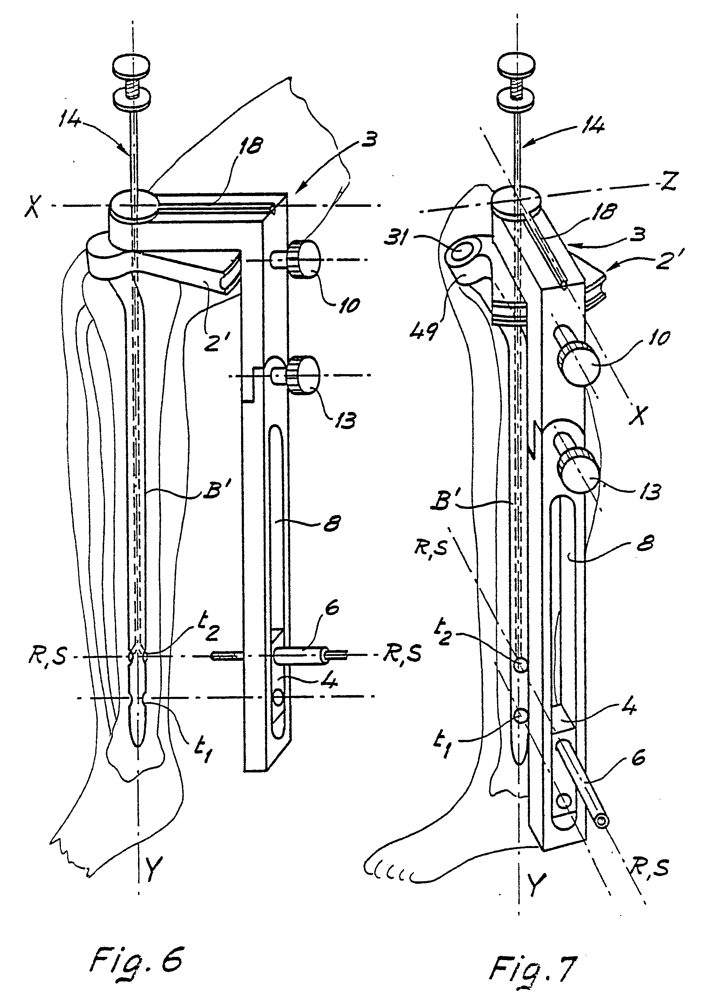

En se référant de nouveau à la figure 3, celle-ci est tracée dans le plan XOZ et sur ce plan a été projetée une section BA de la broche B passant par l'un des trous transversaux TA de celle-ci en supposant que la broche soit parfaitement rectiligne et n'ait subi aucune déformation au cours de l'insertion dans l'os. La figure représente également la projection de la même section indiquée par BB, mais cette fois-ci dans sa position réelle, à savoir lorsque la broche a été déviée par le canal médullaire et en tenant compte de sa forme non rectiligne originale. Afin d'augmenter la clarté de cette figure, la déviation de la broche a été fortement exagérée. Il y a lieu de noter également qu'en toute rigueur cet exemple ne se réfère qu'à une broche de fémur car la broche de tibia (figure 2A) est très cintrée prés de son embouchure ; le support de l'appareil étant alors agencé légèrement différemment (voir figure 7). On reviendra sur ce point par la suite. Cependant, le principe de la mesure résultant de la figure 3 est applicable aussi bien au cas du fémur que celui du tibia.

Il est évident que le centre de la section BA coïncide avec le centre O du système de coordonnées XYZ, le centre de la section BB indiqué par O' étant dévié d'un écart e et la broche elle-même ayant subi une torsion faisant en sorte que le plan diamétral passant par l'axe du trou TA subit une rotation d'un angle $\alpha$ pour se positionner comme indiqué pour le trou TB sur la figure 3. Celle-ci représente également le guide de perçage 6 ainsi que l'esquisse de la section de la branche 3b de l'équerre 3 dans différentes positions qui vont maintenant être expliquées.

Le but de l'opération de repérage consiste en définitive à placer l'axe S-S du guide de perçage (qui se trouve initialement effectivement en coïncidence avec l'axe O-X comme représenté) sur l'axe défini par le trou TB de la broche, c'est-à-dire en coïncidence avec l'axe R-R. Si cette opération peut être réalisée avec précision, le chirurgien sera certain de percer l'os de telle manière que le foret passe dans la broche à travers le trou TB et cela sans qu'il soit nécessaire qu'il voit effectivement ce trou. Il ne sera donc pas nécessaire de procéder à une ouverture sanglante de la chair CH à l'endroit où se trouve le trou TB ni de procéder à une quelconque recherche par rayons X.

Le repérage est réalisé selon l'invention en deux étapes qui consistent respectivement et tout d'abord à placer le guide de perçage 6 de manière que son axe S-S soit situé dans un plan parallèle à l'axe R-R et contenant l'axe O-V, puis à décaler le guide de perçage 6 en translation, de telle manière que les axes R-R et S-S coïncident. On comprend, en examinant la figure 4, que la première étape est réalisée en faisant tourner l'équerre 3 autour de l'axe O-Y (premier degré de liberté du guide de perçage), la seconde étape consistant à casser la genouillère 12a, 12b à l'endroit de l'axe U-U (second degré de liberté), le léger défaut introduit par le fait que cette dernière étape est une rotation étant négligeable, compte tenu des diverses dimensions des organes en jeu (longueur de la broche notamment).

Le décalage angulaire α est mesuré à l'aide des premiers moyens palpeurs 14 qui définissent un plan dans lequel sont situés l'axe de la tige 15 et l'index 18 (voir figures 4 et 5). Le palpeur 16 est capable de faire coïncider ce plan avec l'axe réel R-R du trou TB lorsqu'il est inséré dans la broche B jusqu'à la hauteur du trou. Si cet axe est dévié, l'index 18 ne sera pas situé dans le plan XOY de la figure 4, mais sera incliné de l'angle α recherché par rapport au repère 19 tracé sur la branche 3a de l'équerre 3. Pour faire coïncider l'index 18 avec ce repère 19, il est alors nécessaire de dévisser la vis 10 et de faire tourner l'équerre 3 de l'angle α autour de l'axe O-Y, ce qui place la branche 3b dans la position indiquée en pointillés et référencée 3b' sur la figure 3.

Les premiers moyens palpeurs 14 ont alors fait leur office et le praticien peut les enlever de la broche B.

Il doit les remplacer par les seconds moyens palpeurs 20 dont un mode de réalisation préféré est représenté à la figure 9. Dans le cas représenté, ces moyens comportent une lampe éclair 21 fixée à l'extrémité d'un cathéter 22 qui est insérable dans la broche B et qui peut être alimenté en énergie par un appareil d'alimentation 23 connu en soi relié à la lampe 21 par des conducteurs 24. La lampe éclair 21 est associée de préférence à un réticule 25 que l'on peut insérer dans le bloc coulissant 4 (figure 4) à la place du guide de perçage 6. Pour obtenir le décalage de la distance d de l'axe O-X' (figure 3) vers l'axe R-R, il suffit alors de faire marcher la lampe éclair 21 après l'avoir insérée dans la broche jusqu'à la hauteur du trou TB, de dévisser la vis 13 bloquant la genouillère 12a, 12b et de faire en sorte que le centre du réticule 25 se place au centre du trou TB dont le contour apparaît clairement à travers la chair CH du membre concerné grâce aux éclairs produits par la lampe 21. Une fois la coïncidence obtenue, il suffit de rebloquer la vis 13, d'ôter le réticule 25 et de réinsérer le guide de perçage 6 dans le bloc 4, l'axe S-S de ce guide coïncidant alors exactement avec l'axe R-R du trou TB.

Il est à noter que le processus qui vient d'être décrit est exactement le même qu'il s'agisse de la contention d'une facture du fémur ou du tibia, à la réserve près indiquée plus haut en ce qui concerne le support 2.

On remarquera également que les opérations de repérage sont extrêmement simples et rapides, le chirurgien pouvant les exécuter en l'espace de 5 minutes seulement, alors que jusqu'ici avec un appareil à rayons X, il lui faut approximativement deux heures pour obtenir le même résultat, encore que la précision de la mesure ne soit pas aussi satisfaisante que celle obtenue à l'aide de l'appareil de l'invention.

On va maintenant décrire plus en détail la construction de cet appareil en se reportant aux figures 4 à 7.

Le support 2 (figure 5) comprend une plaque de fixation 26 sur laquelle est ménagée une saillie 26a qui a une forme complémentaire à l'embouchure a1 de la broche B, cette forme n'étant pas de révolution de manière à ce que cette plaque ne puisse être bloquée par simple vissage que dans une seule position radiale par rapport à la broche B. La plaque 26 est solidaire d'une plaque d'appui 26b, par exemple à l'aide d'une fixation à queue d'aronde 27, le bord circulaire de cette plaque d'appui présentant deux flasques latéraux 28a et 28b délimitant une rainure dont le fond est creusé en arrondi (voir figure 5B en 29).

La plaque 26 est percée d'un trou qui est destiné à recevoir la portée intermédiaire 30 d'une vis 31 dont l'une des extrémités 32 consiste en un embout fileté adapté au filetage de la broche B et dont la partie d'extrémité opposée 33 sert de palier de rotation à la branche 3a de l'équerre 3 en étant insérée dans une ouverture 34 de cette branche. La vis 31 est percée d'un trou axial 35 afin de livrer passage aux moyens palpeurs 15 et 20. Enfin, la face d'extrémité de la partie 33 de la vis 31 est creusée d'une cavité hexagonale 36 pour permettre le vissage à l'aide d'une clé à six pans.

La vis de blocage 10 (figure 5A) qui traverse la branche 3b à travers un trou fileté 37 porte à son extrémité un doigt de centrage 38 qui est destiné à venir s'appliquer contre le fond 29 de la rainure délimitée par les flasques 28a et 28b afin d'appliquer l'équerre 3 contre la plaque 26. Ce blocage assure également la solidarisation en rotation de l'équerre par rapport à cette plaque et maintient donc la cohérence des divers organes de l'appareil tout en rendant son démontage extrêmement aisé.

Les premiers moyens palpeurs 14 ont été représentés en détail sur la figure 8 sur laquelle on aperçoit la tige 15, le palpeur 16 et l'index 18 déjà mentionnés ci-dessus. A son extrémité distale, la tige 15 porte deux mâchoires 39a et 39b articulées autour d'un axe 40 et sollicitées vers l'extérieur grâce à un filin 41. Celui-ci traverse la tige 15 et est attaché à une plaquette d'actionnement 42 coulissant sur la tige 15 à l'encontre de l'action d'un ressort de compression 43. Ce ressort prend appui sur une contre-plaquette 44 montée transversalement au bout de la tige 15 et fixe par rapport celle-ci. Ainsi, en saisissant la tige d'une main avec le pouce appuyé sur la contre-plaquette 44 et deux doigts accrochés derrière la plaquette d'actionnement 42, il est possible, en comprimant le ressort 43, de rapprocher les mâchoires 39a et 39b l'une de l'autre en les faisant tourner autour de l'axe 40 afin de faciliter l'insertion des moyens palpeurs 14 dans la broche B. Lorsque les mâchoires arrivent à la hauteur d'un trou t1 ou t2, ce dont le praticien peut s'apercevoir facilement car la longueur de la broche est connue d'avance, il relâche la plaquette 42 et les mâchoires s'introduisent par leurs extrémités arrondies dans le trou correspondant de la broche, ce qui bloque la tige 15, non seulement dans le sens de la longueur, mais également en rotation, les mâchoires écartées 39a et 39b définissant un plan diamétral dans lequel est situé l'axe du trou concerné.

Une plaquette de support 45 est montée coulissante sur la tige 15, tout en étant pourvue de ressorts 47 qui coopèrent avec des stries transversales 48 prévues sur cette tige pour en définir différentes longueurs, chaque position correspondant à une longueur de broche susceptible d'être utilisée par le chirurgien. Par conséquent, au départ, cette plaquette 45 est réglée de telle manière que les moyens palpeurs 14 soient adaptés à la longueur de la broche B utilisée. Les stries 48 sont donc avantageusement associées à une graduation de longueur. La plaquette de support 45 porte l'index 18 ici constitué par une tige rectiligne s'étendant radialement. C'est cette tige qui doit donc être mise en coïncidence avec le repère 19 de la branche 3a.

Il est à noter que la position radiale de l'index 18 correspond au plan défini par les mâchoires 39a et 39b écartées, si bien que lorsque celles-ci sont bloquées dans un trou de la broche, l'index 18 est situé dans un plan dans lequel se trouve également l'axe du trou concerné.

Les figures 6 et 7 montrent le cas d'un repérage lors d'une intervention sur le tibia. Comme les broches de tibia sont pourvues d'un embout incliné (voir la figure 2A), le support de l'appareil utilisé dans ce cas est légèrement différent de celui que l'on vient de décrire. En effet, ce support indiqué par la référence 2' comporte une patte latérale 49 dans laquelle est insérée la vis de fixation 31 (figure 5) ce support comportant un trou de passage pour permettre l'insertion des moyens palpeurs 14, à peu près dans l'alignement de la broche B', ce support définissant, comme dans le cas décrit ci-dessus, le plan de référence YOZ par rapport auquel les autres paramètres de repérage sont définis. Pour le reste, tant sur le plan de sa construction que sur le plan de son fonctionnement, l'appareil des figures 6 et 7 est identique à celui représenté sur la figure 4.

On va maintenant décrire un autre mode de réalisation de la partie 12b de la genouillère qui constitue la branche 3b de l'équerre 3 (figure 10). Dans ce cas, cette partie 12b comporte une règle 50 à section en forme de L, dont les deux ailes sont pourvue de rainures 51 et 52 dans lesquelles est guidé un coulisseau 53 conformé de manière à pouvoir recevoir le guide de perçage 6. Bien entendu, la règle 50 peut être bloquée par rapport à l'autre partie 12a de la genouillère grâce à l'écrou 13. Par ailleurs, il est possible de prévoir une réglette graduée 54 montée de manière amovible sur la tranche de l'une des ailes de la règle 50 et destinée à fournir au praticien une indication de la longueur de la broche utilisée.

Sur les figures 11 et 12, on a représenté respectivement avant et après son réglage, un appareil réalisé suivant un autre mode de réalisation de l'invention. Plus précisément, la partie 12b de la genouillère constituant la branche 3b de l'équerre 3 comporte ici un bloc de réglage 55 qui défile devant une règle qui est à fleur avec la face supérieure de celle-ci. De cette manière, un repère 56 tracé sur ce bloc peut coopérer avec une graduation 57 prévue directement sur la règle.

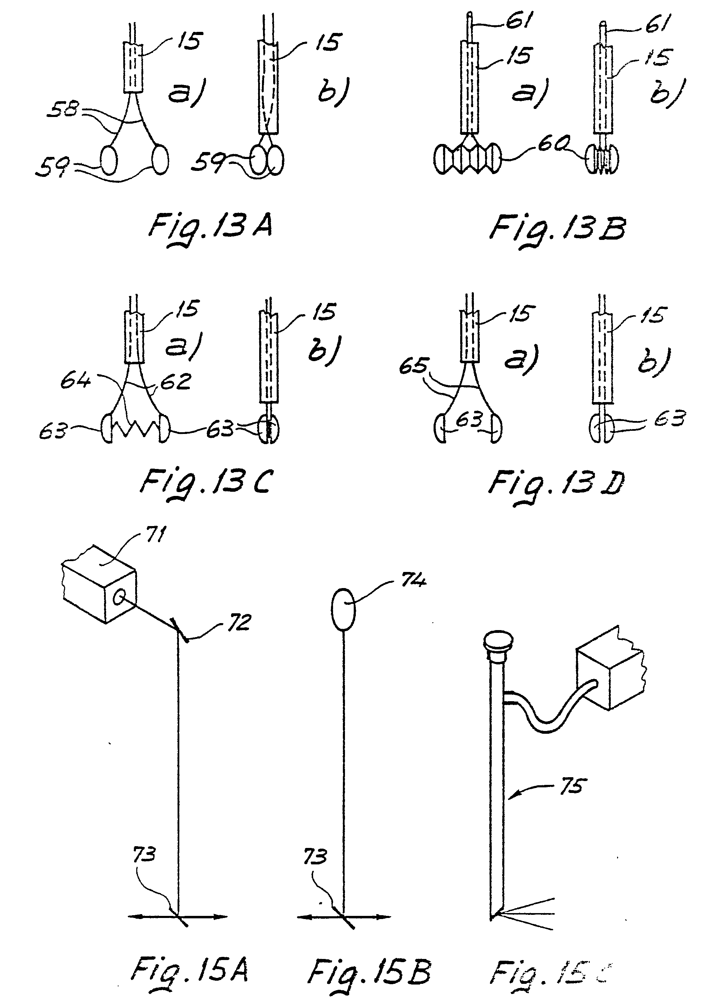

Les figures 13A à 13D montrent plusieurs possibilités de réalisation des premiers moyens palpeurs.

Sur la figure 13A, la tige 15 porte à son extrémité libre deux pattes élastiques 58 qui sont munies à leur extrémité libre de billes 59 capables de pénétrer partiellement dans les ouvertures qui définissent dans chaque broche les trous transversaux de fixation. Les pattes 58 et les billes 59 peuvent être escamotées par traction pour entrer à peu près dans le prolongement du contour de la tige 15 lors de l'insertion des moyens palpeurs dans la broche.

La vue schématique de la figure 13B montre que les premiers moyens palpeurs peuvent comporter également un organe 60 en forme de soufflet pouvant être gonflé ou dégonflé par de l'air insufflé ou évacué d'un tuyau 61 placé dans la tige 15.

La figure 13C montre qu'il est également possible de prévoir au bout de la tige 15 deux pattes de support 62 portant à leur extrémité libre des coupelles 63 sollicitées à l'écartement par un ressort 64. Ces coupelles peuvent être escamotées comme représenté en b sur la figure 13C par traction axiale sur les pattes 62, ce qui comprime le ressort 64.

Dans le cas de la figure 13D, il s'agit également de coupelles 63 qui, dans ce cas sont fixées au bout de tiges élastiques 65 qui les maintiennent écartées, à moins qu'elles ne soient retirées dans la tige 15 par la traction axiale, ce qui efface les coupelles dans le prolongement du contour de cette tige.

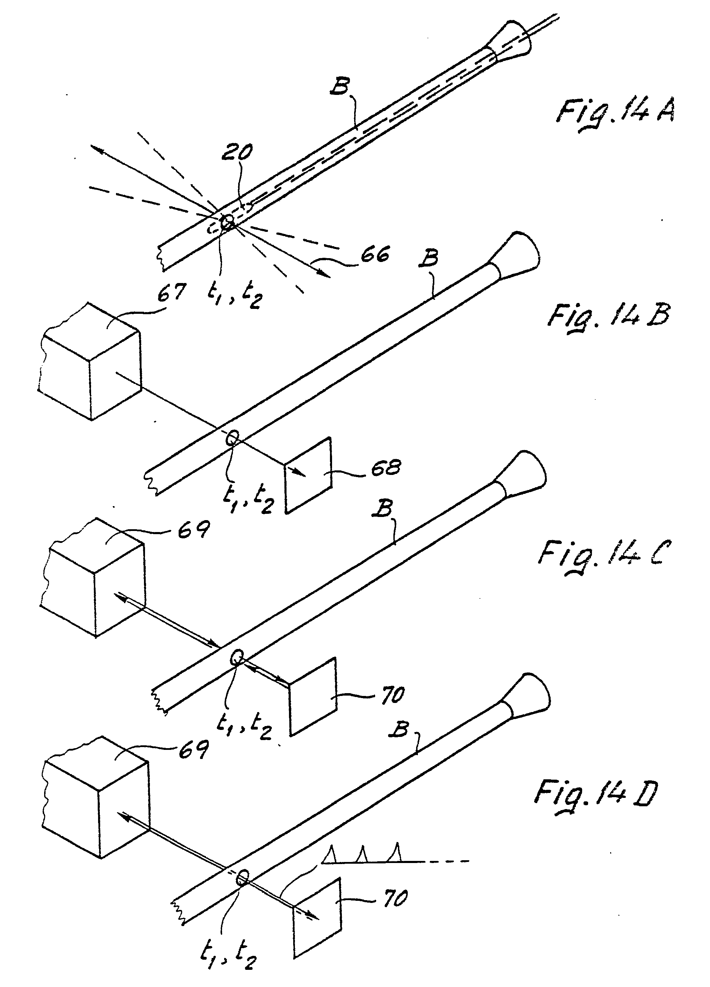

Les figures 14A à 14D et 15A à 15C montrent plusieurs variantes possibles des seconds moyens palpeurs. A la figure 14A on a représenté, à titre de rappel, les moyens palpeurs 20 représentés déjà sur la figure 9. Il s'agit ici donc d'une lampe éclair 21 pouvant émettre un rayonnement lumineux 66 dans la broche B vers l'extérieur, ce rayonnement lumineux étant apparent à travers la chair du membre en traitement, l'effet lumineux étant amélioré par les éclairs fournis par la lampe.

A la figure 14B on a représenté une variante dans laquelle il est prévu à l'extérieur du membre sous traitement une source de rayonnement 67 non traumatisant, cette source rayonnant à travers le trou concerné de la broche B sur un capteur 68 de ce rayonnement, celui-ci pouvant être éventuellement pulsé. Dans ce cas, l'axe du trou peut être trouvé par exemple en détectant une amplitude maximale du rayonnement émis. Le rayonnement lui-même peut être constitué par une onde radio à haute fréquence d'une longueur d'onde choisie de telle manière qu'elle ne soit pas traumatisante pour le patient ou pour le personnel traitant.

A la figure 14C, il s'agit d'un émetteur-récepteur 69 de rayonnement qui envoie son rayonnement sur un réflecteur 70 placé en face. Celui-ci renvoye une partie du rayonnement à travers le trou de la broche pour qu'elle puisse y être détectée.

Enfin, la figure 14D montre que dans le cas d'un réflecteur 70 le rayonnement peut être pulsé ou caractérisé d'une autre manière par une modulation afin d'en améliorer la détection et, partant, la précision du repérage du trou de la broche.

La figure 15A représente encore une autre variante dans laquelle il est prévu une source lumineuse 71 qui peut être un laser par exemple, émettant un rayon lumineux sur un miroir 72 monté dans le support 3 de l'appareil et renvoyant le rayon lumineux sur un autre dispositif réflecteur 73 se trouvant au bout d'une tige (non représentée). Ce dernier dispositif renvoie le rayonnement dans les deux sens à travers le trou de la broche, ce qui permet son repérage, par exemple par un réticule, comme cela a été décrit à propos de la figure 9.

Bien entendu, le laser 71 peut être remplacé par une source lumineuse à éclairs 74 représentée à la figure 15B. Enfin, il est également possible d'utiliser un endoscope classique 75 de la forme représentée sur la figure 15C.

## Revendications

1. Appareil pour repérer in situ les trous transversaux d'une broche creuse (B) implantée dans le canal médullaire (C) d'un os (OS) pour la contention de fragments de celui-ci après une fracture, ladite broche (B) étant pourvue à son extrémité proximale de moyens de fixation pour ledit appareil, tandis que le ou les trous TB à repérer sont prévus au moins à son extrémité distale, cet appareil comprenant :

— un support (2) destiné à être rapporté sur ladite broche (B) à l'aide desdits moyens de fixation, ;

— un guide de perçage (6) définissant un axe (S-S) devant être mis en coïncidence avec l'axe (R-R) du trou (TB) à repérer pour permettre le perçage transversal de l'os exactement en alignement avec ce trou (TB) ;

— une monture (3) solidaire dudit support (2) et portant ledit guide de perçage, ladite monture comprenant au moins une première articulation permettant une rotation dudit guide de perçage autour de ladite broche dans un plan contenant l'axe (R-R) du trou (TB) et une deuxième articulation permettant une translation de l'axe (S-S) du guide de perçage (6) parallèle à lui-même ;

ledit appareil étant caractérisé en ce qu'il comprend en outre :

— des premiers moyens palpeurs (14) destinés à être insérés dans ladite broche en s'orientant en fonction de la direction de l'axe (R-R) du trou (TB), et des premiers moyens indicateurs (18) associés auxdits premiers moyens palpeurs et disposés à l'extérieur de ladite broche pour indiquer l'angle α formé par l'axe (R-R) dudit trou (TB) et l'axe (S-S) dudit guide de perçage, lorsque lesdits premiers moyens palpeurs sont insérés dans ladite broche,

— des seconds moyens palpeurs (20) destinés à émettre un rayonnement électromagnétique à partir dudit trou (TB) vers le guide de perçage et des seconds moyens indicateurs (25) associés audit guide de perçage pour indiquer la distance (d) entre l'axe (R-R) du trou (TB) et l'axe (S-S) du guide de perçage,

— des moyens de réglage pour amener l'axe (S-S) dudit guide de perçage (6) en coïncidence avec l'axe (R-R) dudit trou (TB) en fonction des informations angulaire et de distance fournies successivement par les premiers et seconds moyens palpeurs.

2. Appareil selon la revendication 1 caractérisé en ce que les premiers moyens palpeurs (14) sont fixés à une extrémité d'une tige (15) et lesdits premiers moyens indicateurs (18) sont constitués par un index fixés à l'autre extrémité de la tige (15), ladite tige pouvant être insérée dans ladite broche.

3. Appareil selon l'une quelconque des revendications 1 et 2 caractérisé en ce que les seconds moyens palpeurs (20) comprennent un dispositif (21) émetteur d'un rayonnement électromagnétique non traumatisant capable d'émettre un faisceau à partir du trou à repérer et les seconds moyens indicateurs comportent un dispositif à réticule (25) destiné à être placé sur le guide de perçage et à être centré sur le faisceau de rayonnement perceptible dans ce guide a travers ledit trou, l'os et la chair qui l'entourent, par déplacement du guide de perçage (6).

4. Appareil suivant l'une quelconque des revendications 1 à 3 caractérisé en ce que ladite deuxième articulation comprend des moyens (4, 8) permettant une translation du guide de perçage parallèle à l'axe de la broche et des moyens (12a, 12b) permettant une rotation de l'axe (S-S) du guide de perçage autour d'un axe (U-U) perpendiculaire à ladite translation.

5. Appareil suivant l'une quelconque des revendications 3 et 4 caractérisé en ce que le dispositif émetteur de rayonnement (21) est agencé pour émettre un rayonnement pulsé.

6. Appareil suivant l'une quelconque des revendications 3 à 5 caractérisé en ce que ledit rayonnement, éventuellement cohérent, a la longueur d'onde de la lumière visible.

7. Appareil suivant la revendication 6, caractérisé en ce que ledit dispositif émetteur est une lampe (21) montée à l'extrémité d'un cathéter insérable dans la broche (B).

8. Appareil suivant les revendications 5 et 7 caractérisé en ce que ladite lampe (21) est une lampe éclair.

9. Appareil suivant l'une quelconque des revendications 3 et 4 caractérisé en ce que ledit dispositif émetteur fait partie d'un ensemble émetteur-récepteur (69) placé d'un côté sur l'axe du trou (TB) à repérer et comprenant en outre un dispositif réflecteur (70) placé de l'autre côté du trou sur ce même axe (R-R).

10. Appareil suivant l'une quelconque des revendications 1 à 9 caractérisé en ce que ladite monture (3) présente la forme d'une équerre dont l'une des branches (3a) est montée articulée sur ledit support (2) en s'étendant à peu près perpendiculairement par rapport à ladite broche (B) lorsque l'appareil est en place, en ce que l'autre branche (3b) s'étend à peu près longitudinalement par rapport à cette broche le long de l'os à contenir en étant réalisée comme une genouillère (12a, 12b), l'élément de cette genouillère éloignée de la première branche de l'équerre (3) portant de façon coulissante ledit guide de perçage (6).

11. Appareil suivant l'une quelconque des revendication 2 à 10 caractérisé en ce que lesdits premiers moyens palpeurs (14) comprennent deux éléments de palpage (39a, 39b) montés articulés sur l'extrémité de la tige (15) destinée à être insérée dans la broche, ces éléments étant capables de se bloquer dans les ouvertures de la broche (B) définissant ledit trou transversal (TB) moyennant quoi ladite tige acquiert une position angulaire fixe représentant la direction de l'axe du trou, répercuté sur la position spatiale dudit index (18).

12. Appareil suivant les revendications 10 et 11 caractérisé en ce que ledit index (18) coopère avec un repère (19) tracé sur la première branche (3a) de l'équerre de ladite monture.

13. Appareil pour repérer in situ la direction des axes des trous transversaux d'une broche creuse (B) implantée dans le canal médullaire (C) d'un os (OS) pour la contention de fragments de celui-ci après une fracture, ladite broche (B) étant pourvue à son extrémité proximale de moyens de fixation pour ledit appareil, tandis que le ou les trous transversaux (TB) sont prévus au moins à son extrémité distale, cet appareil comprenant :

— un support (2) destiné à être rapporté sur ladite broche (B) à l'aide desdits moyens de fixation ;

— un guide de perçage (6) définissant un axe (S-S) devant être mis en coïncidence avec l'axe (R-R) du trou (TB) à repérer pour permettre le perçage transversal de l'os exactement en alignement avec ce trou (TB) ;

— une monture (3) solidaire dudit support (2) et portant ledit guide de perçage, ladite monture comprenant une articulation permettant une rotation dudit guide de perçage autour de ladite broche dans un plan contenant l'axe (R-R) du trou (TB) ; ledit appareil étant caractérisé en ce qu'il comprend en outre :

— des moyens palpeurs (14) destinés à être insérés dans ladite broche en s'orientant en fonction de la direction de l'axe (R-R) du trou (TB), et des moyens indicateurs (18) associés auxdits moyens palpeurs et disposés à l'extérieur de ladite broche pour indiquer l'angle α formé par l'axe (R-R) dudit trou (TB) et l'axe (S-S) dudit guide de perçage, lorsque lesdits premiers moyens palpeurs sont insérés dans ladite broche,

— des moyens de réglage pour amener l'axe (S-S) dudit guide de perçage (6) parallèlement à l'axe (R-R) du trou (TB) en fonction de l'information angulaire fournie par lesdits moyens indicateurs.

14. Appareil selon la revendication 13 caractérisé en ce que les moyens palpeurs sont fixés à une extrémité d'une tige (15) et les moyens indicateurs (18) sont constitués par un index fixés à l'autre extrémité de la tige (15), ladite tige pouvant être insérée dans ladite broche.

15. Appareil suivant l'une quelconque des revendications 13 et 14 caractérisé en ce que ladite monture (3) présente la forme d'une équerre dont l'une des branches (3a) est montée articulée sur ledit support (2) en s'étendant à peu près perpendiculairement par rapport à ladite broche (B) lorsque l'appareil est en place, en ce que l'autre branche (3b) s'étend à peu près longitudinalement par rapport à cette broche le long de l'os à contenir en étant réalisée comme une genouillère (12a, 12b), l'élément de cette genouillère éloignée de la première branche de l'équerre (3) portant de façon coulissante ledit guide de perçage (6).

16. Appareil suivant l'une quelconque des revendications 14 et 15 caractérisé en ce que lesdits moyens palpeurs (14) comprennent deux éléments de palpage (39a, 39b) montés articulés sur l'extrémité de la tige (15) destinée à être insérée dans la broche, ces éléments étant capables de se bloquer dans les ouvertures de la broche (B) définissant ledit trou transversal (TB) moyennant quoi ladite tige acquiert une position angulaire fixe représentant la direction de l'axe du trou, répercuté sur la position spatiale dudit index (18).

17. Appareil suivant les revendications 14 et 15 caractérisé en ce que ledit index (18) coopère avec un repère (19) tracé sur la première branche (3a) de l'équerre de ladite monture.

**Claims**

1. Device for locating in situ the through-holes in a hollow pin (B) implanted in the medullary canal (C) of a bone (OS) for retaining the fragments thereof following fracture, said pin (B) being provided at its proximal end with fastening means for said device, whereas the hole or holes (TB) to be located being provided at least at its distal end, said device comprising :

— a support (2) intended to be mounted on said pin (B) utilising said fastening means ;

— a drilling guide (6) defining an axis (S-S) to be brought into coincidence with the axis (R-R) of the hole (TR) to be located so as to enable transversal piercing of the bone exactly in alignment with such hole (TB) ;

— a mouting (3) fixed to said support (2) and bearing said drilling guide, said mounting having at least a first articulating means enabling a rotation of said drilling guide around said pin in a plane containing the axis (R-R) of the hole (TR) and a second articulating means providing a translation of the axis (S-S) of the drilling guide (6) parallel to itself ; said device being characterized in that it further comprises :

— first feeler means (14) intended to be inserted in said pin so as to orient themselves as a function of the direction of axis (R-R) of hole (TB), and first indicating means (18) cooperating with said first feeler means and located outside said pin for indicating the angle α between axis (R-R) of said hole (TB) and the axis (S-S) of said drilling guide, when said first feeler means are inserted in said pin ;

— second feeler means (20) intended to emit an electromagnetic radiation from said hole (TB) to the drilling guide, and second indicating means (25) cooperating with said drilling guide for indicating the distance d between the axis (R-R) of hole (TB) and the axis (S-S) of the drilling guide,

— adjusting means for bringing the axis (S-S) of said drilling guide (6) into coincidence with the axis (R-R) of said hole (TB) as a function of the angular and distance informations successively delivered by the first and second feeler means.

2. Device according to claim 1, characterized in that said first feeler means (14) are secured to one end of a rod (15) and said first indicating means (18) comprise an index secured to the other end of the rod (15), said rod being adapted to be inserted into said pin.

3. Device according to any one of claims 1 and 2, characterized in that the second feeler means (20) comprise an arrangement (21) for emitting a non-traumatising electromagnetic radiation adapted to emit a beam from the hole to be located, and said second indicating means comprise a reticle (25) adapted to be placed on the drilling guide and to be centered on the beam of radiation detectable in said guide from said hole, the bone and surrounding flesh by displacement of the drilling guide.

4. Device according to any of claims 1 to 3, characterized in that said second articulating means comprise means (4, 8) providing a translation of the drilling guide parallel to the axis of the pin, and means (12a, 12b) providing a rotation of the axis (S-S) of the drilling guide around an axis (U-U) perpendicular to said translation.

5. Device according to any one of claims 3 and 4, characterized in that the radiation emitting arrangement (21) is arranged to emit pulsed radiation.

6. Device according to any one of claims 3 to 5, characterized in that said radiation, which may be a coherent radiation, has a wavelength which is in the visible spectrum.

7. Device according to claim 6, characterized in that the emitting arrangement is a lamp (21) mounted on the end of a catheter adapted to be inserted into the pin (B).

8. Device according to claims 5 and 7, characterized in that said lamp (21) is a pulsed flash lamp.

9. Device according to any one of claims 3 and 4, characterized in that said emitting arrangement is part of an emitter-receiver set (69) placed to one side of the axis of the hole (TB) to be located and further including a reflector means (70) placed on the other side of the hole on the same axis (R-R).

10. Device according to any one of claims 1 to 9, characterized in that said mounting (3) takes the form of a corner angle a first arm (3a) of which is mounted articulated onto said support (2) and extends substantially perpendicularly from the pin (B) when the device is in place, in that the other arm (3b) extends substantially longitudinally relative to the pin along the bone to be retained and being formed as an articulated lever (12a, 12b) in which the element thereof remote from the first arm of the corner angle (3) bears said drilling guide (6) in a manner permitting sliding thereof.

11. Device according to any one of claims 2 to 10, characterized in that said first feeler means (14) comprise two sensing elements (39a, 39b) mounted articuled on the end of the rod (15) intended for insertion into the pin, said elements being adapted to be arrested in the openings of the pin (B) defining the through-hole (TB) whereby said rod assumes a fixed angular position representative of the direction of the hole axis and reflected in the spatial position of said index (18).

12. Device according to claims 10 and 11, characterized in that the index (18) cooperates with a mark (19) drawn on the first arm (3a) of the corner angle of said mounting.

13. Device for locating in situ the direction of the axis of the through-holes in a hollow pin (B) implanted in the medullary canal (C) of a bone (OS) for retaining the fragments thereof following fracture, said pin (B) being provided at its proximal end with fastening means for said device, whereas the hole or holes (TB) to be located being provided at least at its distal end, said device comprising :

— a support (2) intented to be mounted on said pin (B) utilising said fastening means ;

— a drilling guide (6) defining an axis (S-S) to be brought into coincidence with the axis (R-R) of the hole (TR) to be located so as to enable transversal piercing of the bone exactly in alignment with such hole (TB) ;

— a mounting (3) fixed to said support (2) and bearing said drilling guide, said mounting comprising an articulation means enabling a rotation of said drilling guide arount said pin in a plane containing the axis (R-R) of the hole (TR) ; said device being characterized in that it further comprises :

— feeler means (14) intended to be inserted in said pin so as to orient themselves as a function of the direction of axis (R-R) of hole (TB), and indicating means (18) cooperating with said feeler means and located outside said pin for indicating the angle between axis (R-R) of said hole (TB) and the axis (S-S) of said drilling guide, when said feeler means are inserted in said pin,

— adjusting means for bringing the axis (S-S) of said drilling guide (6) parallel to the axis (R-R) of said hole (TB) as a function of the angular information delivered by said feeler means.

14. Device according to claim 13, characterized in that the feeler means are secured to one end of a rod (15) and the indicating means (18) comprise an index secured to the other end of the rod (15), said rod being adapted to be inserted into said pin.

15. Device according to any one of claims 13 and 14, characterized in that said mounting (3) takes the form of a corner angle a first arm (3a) of which is mounted articulated onto said support (2) and extends substantially perpendicularly from the pin (B) when the device is in place, in that the other arm (3b) extends substantially longitudinally relative to the pin along the bone to be retained and being formed as an articulated lever (12a, 12b) in which the element thereof remote from the first arm of the corner angle (3) bears said drilling guide (6) in a manner permitting sliding thereof.

16. Device according to any one of claims 14 and 15, characterized in that said feeler means (14) comprise two sensing elements (39a, 39b) mounted articulated on the end of the rod (15) intended for insertion into the pin, said elements being adapted to be arrested in the opening of the pin (B) defining the trough-hole (TB) wherey said rod assumes a fixed angular position representative of the direction of the hole axis and reflected in the spatial position of said index (18).

17. Device according to claims 14 and 15, characterized in that the index (18) cooperates with a mark (19) drawn on the first arm (3a) of the corner angle of said mouting.

**Patentansprüche**

1. Gerät zum Auffinden in situ der Querbohrungen eines im Markkanal (C) eines gebrochenen Knochens (OS) für das Zusammenhalten der Knochenfragmente implantierten hohles Stiftes (B), wobei der genannte Hohlstift an seinem rumpfseitigen Ende über Befestigungsmittel für das genannte Gerät verfügt, während die aufzufindenden Bohrungen (TB) zumindest am gegenüberliegenden Stiftende vorgesehen sind, bestehend aus :

— einem auf dem genannten Stift (B) mittels der genannten Befestigungsmittel aufzusetzenden Halter (2) ;

— einer Bohrbuchse (6), die eine mit der Achse (R-R) der aufzufindenden Bohrung (TB) deckungsgleich auszurichtende Achse (S-S) definiert, um das Querbohren des Knochens in genauer Fluchtung zu dieser Bohrung (TB) zu gestatten ;

— einem mit dem genannten Halter fest verbundenen und die genannte Bohrbuchse tragenden Bügel (3), der mindestens ein erstes Gelenk umfasst, welches eine Drehung der genannten Bohrbuchse um den genannten Stift in einer die Achse (R-R) der Bohrung (TB) umfassenden Ebene gestattet, und ein zweites Gelenk, welches eine Parallelverschiebung der Achse (S-S) von Bohrbuchse (6) gestattet ;

gekennzeichnet dadurch, dass das Gerät ferner umfasst :

— erste Tastmittel (14), die in den genannten Stift in Abhängigkeit der Richtung der Achse (R-R) von Bohrung (TB) winkelgerecht eingesetzt werden, und erste, den genannten ersten Tastmitteln zugeordnete und aussen an dem genannten Stift angeordnete Anzeigemittel (18) zur Anzeige des von Achse (R-R) der genannten Bohrung (TB) und von Achse (S-S) der genannten Bohrbuchse gebildeten Winkels α, wenn die genannten ersten Tastmittel in den genannten Stift eingesetzt sind,

— zweite Tastmittel (20) zur Emission einer elektromagnetischen Strahlung ab der genannten Bohrung (TB) zur Bohrbuchse und zweite, der genannten Bohrbuchse zugeordnete Anzeigemittel (25) zur Anzeige des Abstands (d) zwischen Achse (R-R) der Bohrung (TB) und Achse (S-S) der Bohrbuchse,

— Einstellmittel, um Achse (S-S) der genannten Bohrbuchse (6) in Abhängigkeit der nacheinander von den ersten und zweiten Tastmitteln gelieferten Winkel- und Längeninformationen in Deckungsgleichheit mit Achse (R-R) der genannten Bohrung (TB) zu bringen.

2. Gerät gemäss Anspruch 1, dadurch gekennzeichnet, dass die ersten Tastmittel (14) am Ende einer Stange (15) befestigt sind und die genannten ersten Anzeigemittel (18) aus einer am anderen Ende der Stange (15) befestigten Zeigermarke bestehen, wobei die genannte Stange in den genannten Stift eingesetzt werden kann.

3. Gerät gemäss einem beliebigen der Ansprüche 1 und 2, dadurch gekennzeichnet, dass die zweiten Tastmittel (20) eine Sendevorrichtung (21) für eine nicht verletzende elektromagnetische Strahlung umfassen, die ab der aufzufindenden Bohrung einen Strahl aussenden kann und die zweiten Anzeigemittel eine Empfangsvorrichtung (25) umfassen, die auf der Bohrbuchse angeordnet ist und auf das in dieser Buchse durch die genannte Bohrung, den Knochen und das diesen ihn umgebende Fleischgewebe hindurch wahrnehmbare Strahlenbündel durch Verstellen der Bohrbuchse (6) zentriert werden kann.

4. Gerät gemäss einem beliebigen der Ansprüche 1 bis 3, dadurch gekennzeichnet, dass das genannte zweite Gelenk Mittel (4, 8) umfasst, die eine Parallelverschiebung der Bohrbuchse zur Achse des Stiftes gestatten und Mittel (12a, 12b), die eine Drehung der Achse (S-S) der Bohrbuchse um eine rechtwinklig zur genannten Verschiebung verlaufende Achse (U-U) gestatten.

5. Gerät gemäss einem beliebigen der Ansprüche 3 und 4, dadurch gekennzeichnet, dass die Strahlensendevorrichtung (21) für eine pulsierte Strahlung ausgelegt ist.

6. Gerät gemäss einem beliebigen der Ansprüche 3 bis 5, dadurch gekennzeichnet, dass die genannte, u. U. kohärente Strahlung die Wellenlänge des sichtbaren Lichts aufweist.

7. Gerät gemäss Anspruch 6, dadurch gekennzeichnet, dass die genannte Sendevorrichtung eine am Ende eines unlösbar mit dem Stift (B) verbundenen Katheters angeordnete Lampe (21) ist.

8. Gerät gemäss den Ansprüchen 5 und 7, dadurch gekennzeichnet, dass die genannte Lampe (21) eine Blitzlampe ist.

9. Gerät gemäss einem beliebigen der Ansprüche 3 und 4, dadurch gekennzeichnet, dass die genannte Sendevorrichtung Bestandteil einer Sende-Empfänger-Gruppe (69) ist, die einerseits auf der Achse der festzustellenden Bohrung (TB) angeordnet ist und ferner eine Reflektorvorrichtung (70) umfasst, die auf der anderen Seite der Bohrung auf derselben Achse (R-R) angeordnet ist.

10. Gerät gemäss einem beliebigen der Ansprüche 1 bis 9, dadurch gekennzeichnet, dass der genannte Bügel (3) die Form eines Winkels hat, dessen einer Schenkel (3a) gelenkig auf dem genannten Halter (2) montiert ist und dabei etwa rechtwinklig zum genannten Stift (B) verläuft, wenn das Gerät an Ort und Stelle ist, und dass der andere Schenkel (3b) etwa längs zu diesem Stift entlang dem zusammenzuhaltenden Knochen verläuft und wie eine Kniekappe (12a, 12b) ausgebildet ist, wobei das vom ersten Schenkel des Winkels (3) entfernte Element der Kniekappe die genannte Bohrbuchse (6) verschiebbar trägt.

11. Gerät gemäss einem beliebigen der Ansprüche 2 bis 10, dadurch gekennzeichnet, dass die genannten ersten Tastmittel (14) zwei Tastelemente (39a, 39b) umfassen, die gelenkig an dem in den Stift einzuführenden Ende der Stange (15) montiert sind, wobei sich diese Elemente in den Offnungen des Stiftes (B) festsetzen können, die die genannte Querbohrung (TB) definieren, so dass die genannte Stange eine feste Winkellage erhält, die die Richtung der Bohrungsachse darstellt und die räumliche Lage der genannten Zeigermarke (18) bestimmt.

12. Gerät gemäss den Ansprüchen 10 und 11, dadurch gekennzeichnet, dass die genannte Zeigermarke (18) mit einer Markierung (19) zusammenarbeitet, die auf dem ersten Schenkel (3a) des Winkels des genannten Bügels angebracht ist.

13. Gerät zum Auffinden in situ der Achsrichtungen der Querbohrungen eines im Markkanal (C) eines gebrochenen Knochens (OS) für das Zusammenhalten der Fragmente implantierten hohles Stiftes (B), wobei der genannte Hohlstift an seinem rumpfseitigen Ende über Befestigungsmittel für das genannte Gerät verfügt, während die aufzufindenden Bohrungen (TB) zumindest am gegenüberliegenden Stiftende vorgesehen sind, bestehend aus :

— einem auf dem genannten Stift (B) mittels der genannten Befestigungsmittel aufzusetzenden Halter (2) ;

— einer Bohrbuchse (6), die eine mit der Achse (R-R) der aufzufindenden Bohrung (TB) deckungsgleich auszurichtende Achse (S-S) definiert, um das Querbohren des Knochens in genauer Fluchtung zu dieser Bohrung (TB) zu gestatten ;

— einem mit dem genannten Halter fest verbundenen und die genannte Bohrbuchse tragenden Bügel (3), der ein Gelenk umfasst, welches eine Drehung der genannten Bohrbuchse um den genannten Stift in einer die Achse (R-R) der Bohrung (TB) umfassenden Ebene gestattet, gekennzeichnet dadurch, dass das Gerät ferner umfasst :

— Tastmittel (14), die in den genannten Stift in Abhängigkeit der Richtung der Achse (R-R) von Bohrung (TB) winkelgerecht eingesetzt werden, und den genannten Tastmitteln zugeordnete und aussen an dem genannten Stift angeordnete Anzeigemittel (18) zur Anzeige des von Achse (R-R) der genannten Bohrung (TB) und von Achse (S-S) der genannten Bohrbuchse gebildeten Winkels α, wenn die genannten Tastmittel in den genannten Stift eingesetzt sind,

— Einstellmittel, um Achse (S-S) der genannten Bohrbuchse (6) in Abhängigkeit der von den Tastmitteln gelieferten Winkelinformation parallel zur Achse (R-R) der genannten Bohrung (TB) zu justieren.

14. Gerät gemäss Anspruch 13, dadurch gekennzeichnet, dass die Tastmittel am Ende einer Stange (15) befestigt sind und die Anzeigemittel (18) aus einer am anderen Ende der Stange (15) befestigten Zeigermarke bestehen, wobei die genannte Stange in den genannten Stift eingesetzt werden kann.

15. Gerät gemäss einem beliebigen der Ansprüche 13 und 14, dadurch gekennzeichnet, dass der genannte Bügel (3) die Form eines Winkels hat, dessen einer Schenkel (3a) gelenkig auf dem genannten Halter (2) montiert ist und dabei etwa rechtwinklig zum genannten Stift (B) verläuft, wenn das Gerät an Ort und Stelle ist, und dass der andere Schenkel (3b) etwa längs zu diesem Stift entlang dem zusammenzuhaltenden Knochen verläuft und wie eine Kniekappe (12a, 12b) ausgebildet ist, wobei das vom ersten Schenkel des Winkels (3) entfernte Element der Kniekappe die genannte Bohrbuchse (6) verschiebbar trägt.

16. Gerät gemäss einem beliebigen der Ansprüche 14 und 15, dadurch gekennzeichnet, dass die genannten Tastmittel (14) zwei Tastelemente (39a, 39b) umfassen, die gelenkig an dem in den Stift einzuführenden Ende der Stange (15) montiert sind, wobei sich diese Elemente in den Öffnungen des Stiftes (B) festsetzen können, die die genannte Querbohrung (TB) definieren, so dass die genannte Stange eine feste Winkellage erhält, die die Richtung der Bohrungsachse darstellt und die räumliche Lage der genannten Zeigermarke (18) bestimmt.

17. Gerät gemäss den Ansprüchen 14 und 15, dadurch gekennzeichnet, dass die genannte Zeigermarke (18) mit einer Markierung (19) zusammenarbeitet, die auf dem ersten Schenkel (3a) des Winkels des genannten Bügels angebracht ist.

EP 0 187 283 B1

Fig.1

Fig.1A

Fig.2

Fig.2A

Fig. 3

Fig.4

Fig.5

Fig.5A

Fig.5 B

4

Fig.6

Fig.7

Fig.8

Fig.9

Fig.10

Fig. 11

Fig. 12

EP 0 187 283 B1

Fig. 13 A

Fig. 13 B

Fig. 13 C

Fig. 13 D

Fig. 15 A

Fig. 15 B

Fig. 15 C

8

Fig.14 A

Fig. 14 B

Fig. 14 C

Fig. 14 D